# EUROPEAN PATENT SPECIFICATION

(11) **EP 1 924 248 B1**
(45) Date of publication and mention of the grant of the patent: **22.10.2014**
(21) Application number: 05856290.1
(22) Date of filing: 30.12.2005
(51) Int. Cl.: A61K 9/51, A61K 38/28

(54) **PH SENSITIVE NANOPARTICLE FORMULATION FOR ORAL DELIVERY OF PROTEINS/PEPTIDES**
PH-EMPFINDLICHE NANOTEILCHENFÖRMIGE FORMULIERUNG ZUR ORALEN ABGABE VON PROTEINEN/PEPTIDEN
FORMULATION DE NANOPARTICULES SENSIBLES AU PH POUR L'ADMINISTRATION ORALE DE PROTÉINES/PEPTIDES

(30) Priority: 15.09.2005 IN DE24992005
(43) Date of publication of application: 28.05.2008
(73) Proprietor: Council of Scientific and Industrial Research, New Delhi 110001 (IN)
(72) Inventor: SHARMA, Chandra, Prakash, Thiruvananthapuram, Kerala (IN); MANNEMCHERRIL, Ramesan, Rekha, Thiruvananthapuram, Kerala (IN)
(74) Representative: Biggi, Cristina
(86) International application number: PCT/IN2005/000460
(87) International publication number: WO 2007/032018

(56) References cited:
- WO-A-97/47323
- FR-A- 2 854 072
- US-A- 5 804 212
- US-A1- 2002 192 235
- AIEDEH K ET AL: "CHITOSAN MICROCAPSULES AS CONTROLLED RELEASE SYSTEMS FOR INSULIN" JOURNAL OF MICROENCAPSULATION, TAYLOR AND FRANCIS, BASINGSTOKE, GB, vol. 14, no. 5, September 1997 (1997-09), pages 567-576, XP000659514 ISSN: 0265-2048
- PONCELET D ET AL: "PRODUCTION OF ALGINATE BEADS BY EMULSIFICATION/INTERNAL GELATION. II. PHYSICOCHEMISTRY" APPLIED MICROBIOLOGY AND BIOTECHNOLOGY, SPRINGER VERLAG, BERLIN, DE, vol. 43, no. 4, 1995, pages 644-650, XP008030881 ISSN: 0175-7598
- HARI P R ET AL: "CHITOSAN/CALCIUM-ALGINATE BEADS FOR ORAL DELIVERY OF INSULIN" JOURNAL OF APPLIED POLYMER SCIENCE, JOHN WILEY AND SONS INC. NEW YORK, US, vol. 59, no. 11, 1996, pages 1795-1801, XP002055072 ISSN: 0021-8995

## Description

### Field of the invention

The present invention relates to a pH sensitive nanoparticulate delivery system for the administration of peptide hormones and drugs. In particular the present invention relates to oral insulin administration.

### Background of invention

Diabetic mellitus is a common endocrine disorder, which poses a serious healthcare challenge. The global prevalence of diabetes is estimated to increase from 4% in 1995 to 5.4% by the year 2025. The WHO predicted that the major burden would occur in the developing countries. There will be a 42% increase from 51 to 72 million in the developed countries and 170% increase from 84 to 228 million in the developing countries. Countries with the largest number of diabetic people are and will be India, China and United States in the year 2025 (King H, Aubert RE and Herman WH., Diab. Care 1998: 21, 1414-1431).

Diabetes mellitus is a heterogeneous disorder characterized by varying degrees of insulin resistance and insulin deficiency, which leads to disturbance in glucose homeostasis. The disease if uncontrolled, is characterized by high blood glucose levels, polydipsia, polyuria, polyphagia, feeling of tiredness, blurred vision and weight gain or weight loss.Diabetes mellitus is classified into two major forms; Type I (IDDM) characterized by insulin deficiency resulting from pancreatic beta-cell destruction mediated by autoimmune disorder and Type II (NIDDM) is generally characterized by peripheral insulin resistance and relative insulin deficiency, which may range from predominant insulin secretory defect with insulin resistance.

Insulin is the most important drug for diabetic therapy and insulin administration is the treatment for all Type-I diabetic patients and many Type II diabetic patients. Especially for type I diabetic patients the only treatment currently available is taking exogenous insulin injections.

Efforts for developing oral insulin delivery system are one of the most active research areas for the past many years. At present the diabetic patients who are dependent on insulin for normal life have to take multiple subcutaneous injections a day, which is a painful ordeal. Moreover daily injections can lead to infections and thereby other relat4ed complications. So many efforts are being taken worldwide to realize an alternative insulin delivery route other than parenteral. Being protein insulin cannot be given orally which is the most accepted route of drug intake. The major hurdles in getting insulin into the systemic circulation orally are digestive acids, enzymes and poor absorption via the intestinal wall. Packaging the drugs along with protease inhibitors or giving protective coating could enable protein-based drugs to survive the intestinal conditions, but it cannot aid them in crossing the gut lining. To overcome these obstacles various polymers are tried for developing micro and nanoparticles as oral peptide carriers. It is reported that nanoparticles can easily reach the systemic circulation from the intestine via Peyer's patches.

In view of the above facts we have developed a fatty acid-polymeric nanoparticle based oral insulin formulation, which takes care of both the hurdles faced in oral protein delivery. The polymer that is encapsulating the insulin is pH sensitive. The lipid-polymer complex protects the insulin from the harsh gastro-intestinal environment and its nanomeric size helps to cross the intestinal barrier efficiently. This nanoparticles has shown to have sustained - release property. The nanoparticles are prepared by water-in-oil emulsion mechanism. The polymers used in nanoparticle preparations include alginates, derivatised chitosan, derivatised pullulan, gellan, xanthan. The preparation medium is oil. The oils used are edible grade coconut oil, groundnut oil, rice bran oil, olive oil, palm kernel oil, palm oil etc individually as well as blends of various ratios of these oils or mixture, of the essential contents of the oil namely fatty acids.

### Objectives of the invention

The main objective of the present invention is to provide a nano particle formulation for delivery system for the administration of peptide hormones and drugs.

Yet another object is to provide a nano particle formulation for an oral insulin administration.

Yet another object is to provide a process for the preparation of a nano particle formulation for delivery system for the administration of peptide hormones and drugs.

Still another object is to provide a fatty acid-polymeric nanoparticle based oral insulin formulation, which could take care of the hurdles faced in oral protein delivery

### Summary of the invention

Accordingly the present invention provides a novel pH sensitive nano particle formulation comprising

| | |
|---|---|
| a) polymer | 4-6% (w/w) |
| b) fatty acid | 25-50% (w/w) |
| c) surfactant | 0.5-2.0%(w/w) |
| d) protein | 0.5-2.5%(w/w) (1mg of protein contains 25 IU) |
| e) cross linking agent | 0.02-0.55% (w/w) |
| f) water content | 50-75% (w/w) |

In an embodiment of the present invention the pH sensitive nanoparticle formulation has the following characteristics:
a) having the particle size distribution as prepared for TEM analysis is in the range of 30-100nm (>90%),
b) activity of the loaded insulin in the said formulation is 100% and is stable for a period of 5-7 months, at a temperature of about 4°C,
c) the said formulation is capable of lowering blood glucose level by over 50% in diabetic rats when administered with a dose of 6 IU/200 gm body weight,
d) bioavailability/absorption of protein is about 27% in the rat model when the said formulation is administered in the above said model.

In yet another embodiment of the present invention the polymer used is selected from the group consisting of chitosin, alginate, pullulan, chitosan, gellan, xanthan, poly methacrylic acid and their derivatives thereof.

In yet another embodiment the protein used is selected from insulin and harmones.

In yet another embodiment the cross linking agent used is selected from the group consisting of ZnCl₂, CaCl₂, glutaraldehyde and a mixture thereof.

In yet another embodiment the fatty acid used is selected from the group consisting of lauric acid, oleic acid myristic acid, palmitic acid and linoleic acid.

In another embodiment the oil used is selected from edible grade oil, groundnut oil, rice bran oil, olive oil, palm kernel oil, palm oil and a mixture thereof.

In yet another embodiment the nanoparticle formulation is useful for oral delivery system in a body for the administration of peptide hormones and drugs.

In yet another embodiment the said nanoparticle formulation reduces blood glucose level by over 50% in diabetic rats when administered with a dose of 6 IU/200 gm body wt.

The present invention further provides a process for the preparation of novel pH sensitive nano particle formulation comprising

| | |
|---|---|
| a) polymer | 4-6% (w/w) |
| b) fatty acid | 25-50% (w/w) |
| c) surfactant | 0.5-2.0%(w/w) |
| d) protein | 1.0-2.5%(w/w) (1mg of protein contains 25 IU) |
| e) cross linking agent | 0.02-0.3% (w/w) |
| f) water content | 50-75% (w/w) |

the said process comprises the steps of:
a) preparing a solution of 0.1-0.9% polymer and 40-400IU/mL protein (20% v/v) in water, followed by stirring the above said solution mixture, for a period of about 1 hr,
b) preparing an oil suspension of 0.2-1.0% surfactant and 0.5-2.0% HCl (0.1N) in oil or fatty acid, under stirring at 600-700 rpm, over a period of 10-20 min, at a temperature of 20-25°C,
c) preparing a cross linking solution of 0.03-0.3% (w/w) cross linking agent in oil or fatty acid, under stirring vigorously over a period of about 1 hr,
d) adding soluble polymer -protein solution mixture obtained in step (a) to an oil suspension obtained in step (b) under stirring, for a period of about 1 hr,
e) adding drop wise cross linking solution obtained in step (c) to a solution mixture obtained in step (d), under stirring, and continuing the stirring for a period of about 30 minutes, at 6000-7000 rpm , at a temperature of 30-35°C,
f) filtering the above said solution mixture through 100 nm micro filter, followed by centrifugation at about 10,000 rpm, for a period of 20-30 min and draining the supernatant to obtain the desired nano particle formulation.

In yet another embodiment the polymer used is selected from chitosin, algenate, pullulan, chitosan, gellan, xanthan, poly methacrylic acid and their derivatives thereof

In yet another embodiment the protein used is selected from insulin and harmones.

In yet another embodiment the cross linking agent used is selected from the group consisting of ZnCl₂, GaCl₂, glutaraldehyde and a mixture thereof.

In yet another embodiment the fatty acid used is selected from the group consisting of lauric acid, oleic acid palmitic acid, myristic acid and linoleic acid.

In yet another embodiment the oil used is selected from edible grade oil, groundnut oil, rice bran oil, olive oil, palm kernel oil, palm oil and a mixture thereof.

In still another embodiment the said pH sensitive nano particle formulation obtained has the following characteristics:
a) having the particle size distribution as prepared for TEM analysis is in the range of 30-100nm (>90%),
b) activity of the loaded insulin in the said formulation is 100% and is stable for a period of 5-7 months, at a temperature of about 4°C,
c) the said formulation is capable of lowering blood glucose level by over 50% in diabetic rats when administered with a dose of 6 IU/200 gm body weight,
d) bioavailability/absorption of protein is about 27% in the rat model when the said formulation is administered in the above said model

### Brief description of the drawings:

Figure 1. Diabetic control, placebo and oral insulin formulation (at a dose of 3 and 6 IU/ 200 gm body weight of diabetic rat).
Figure 2. Effect of formulation on normal pigs and also the effect of formulation during glucose infusion (i.v).
Figure3.. Effect of oral insulin formulation on fasting diabetic pigs at doses 9 & 11 IU/ kg body weight.
Figure 4. Effect of oral insulin formulation at a dose of 20 IU/kg body weight on BGL of diabetic pigs under fed conditions.
Figure 5. Nanoparticles in Peyer's patches and villi.
Figure6. Transmission Electron Photomicrograph of insulin loaded polymeric nanoparticles along with the size calculator.

### Detail Description of the invention

The nanoparticles developed by this process are fatty acid nanoparticles and a polymer is used as a stabilizer and also to incorporate pH sensitivity so that these particles shrink in the gastric acidic pH thereby protecting the incorporated insulin. These particles being also hydrophobic in nature and by virtue of their small size get absorbed through the intestinal cell wall and Peyer's patches. These nanoparticles are novel and unique in the sense that polymer content is only 0.03-0.06g/g product and the polymer is hydrophilic in nature. Due to its hydrophilic nature the drug is incorporated during the preparation process itself and a hydrophobic coating of fatty acids is also developed. Due to its hydrophobic nature the particles are readily absorbed into the systemic circulation from the intestine via villi and Peyer's patches. Only specific oil used in this preparation exhibit this property as trials were made from the usage of other oils in our laboratory, which had similar fatty acid composition. The major component of these nanoparticles is fatty acids.
The polymer component is only <0.06g/g of the final product.

The fatty acid component and polymer part is crosslinked using crosslinking agents which results in the formation of stable nanoparticles which is possessing both hydrophobicity and hydrophilicity.

The insulin-loaded nanoparticles developed were found to be very efficient.
The loading efficiency ranges from 50 to 80 % depending on the nature of insulin solution used for the particle preparation.

The size of the particles was determined by Transmission Electron Microscopy and was in the range of 30-100 nm, majority having size less than 100 nm (Figure attached)

The particles show pH sensitivity; at gastric pH the particles will shrink and this protects the insulin from the acidic environment and the digestive enzymes.

In the intestine, being nanoparticles they get readily absorbed by the Peyer's patches in the ileum region (Figure attached). They get absorbed by villi also.
The activity of the loaded insulin was determined by ELISA and found that the loaded insulin retained 100% activity. The efficacy of this formulation was studied in vivo using rat model. The formulation was capable of lowering the blood glucose level by over 50% in diabetic rats with a dosage of 6 IU/200 gm body weight. The effect sustained for about 11-13 hours from the onset.

This is also demonstrated in pig model that the insulin-loaded nanoparticle when given orally is capable of reducing the blood glucose levels.

Stability: Stability studies of biological activity of loaded insulin in nanoparticles have been performed upto six months and it has been observed to be 100% bioactive. The stability data in the literature does not exist from other laboratories to our knowledge on search. Further studies will be performed for longer duration.
Absorption: Various groups have demonstrated the bioabsorption of insulin from oral insulin delivery systems with varied results (Ref: 1-3) from 4 % to 21% in various species. Our preliminary studies in rat model suggest up to 27% absorption which is supposed to be enhanced in higher species. Our formulation seems to be providing enhanced absorption of insulin via absorption of nanoparticles.

| **Oral insulin** | **Animal species** | **Bioavailability** |
|---|---|---|
| **Present invention** | **Rat** | **27.0%** |
| **Ref:1** | **Dogs** | **8.0%** |
| **Ref:2** | **Rats** | **4.2%** |
| **Ref:3** | **Rats and dogs** | **21.0%** |

| | | |
|---|---|---|
| **Ref: 1. J. Gordon Still. Diabetes/Metabolism research and Reviews. 2002(18): S29-37.** **Ref: 2. Lowmann, A.M. Journal of Pharmaceutical Sciences. Vol.88 (9). 1999.** **Ref: 3. http://www.che.utexas.edu/research/biomat/research/delivery.htm** | | |

In accordance with the present invention the pharmaceutical composition is provided comprising insulin encapsulated into a novel unique nanoparticle carrier comprising of fatty acids and polymer. The major component is fatty acids, which comes from the preparation medium, which is oil, and the polymer component in which insulin is encapsulated. The advantage of the polymer is in providing protection as well as stability to the encapsulated insulin. This ensures the 100% biological activity of the loaded insulin in the nanoparticle. The anionic class of polymers such as xanthan, alginic acid, gellan and anionic derivatives of chitosan and pullulan etc. can be used.
**The constituents of the formulation** are polymer 0.1-0.9% (w/v), insulin solution 40-400 IU/ml 20.0% (v/v), oil 70-80%, surfactant 0.2 -1.0% (v/v), N/10 HCl 0.5-2.0% (v/v), CaCl₂ 2H₂O (0.02-0.2%), Zn Cl₂ (0.010-0.1%).

The polymer is dissolved in the insulin by stirring using magnetic stirred at a low speed. The stirring was done for an hour. To seventy % of the oil or the fatty acid component, surfactant and HCl is added and stirred at 6000 rpm in a suitable preparation container using a high-speed stirrer using a half-moon paddle stirrer. After stirring for 15 minutes the insulin-polymer solution was added slowly without stopping the stirring. The stirring was continued for two hours at the same rpm. Both CaCl₂ 2H₂O and Zn Cl₂ are dispersed in the remaining oil or fatty acid component by stirring vigorously using a magnetic stirrer. This dispersion is added after two hours to the oil-insulin-polymer mixture very slowly without stopping the stirring, which is then continued for again 30 minutes to 1 hour at the same speed. The temperature of the system should be maintained at 35 to 36°C. The suspension is then filtered through a 100 nm micro filter and centrifuged at 10,000 rpm for twenty minutes. The supernatant is drained off and the pellet obtained is stored at 4-8°C.

The following examples are given by the way of illustration and therefore should not be construed to limit the scope of the invention.

### Example 1

**Materials**

| | |
|---|---|
| Derivatised chitosan - 0.1- 0.9% optimized to get < 0.06g/g of the final product | |
| Insulin solution- | 10- 20% |
| Palmoil:Coconut oil 50:50- | 75-80% |
| Sorbitan monooleate- | 0.3- 0.5% |
| CaCl₂ 2H₂O- | 0.15 - 0.2% |

The derivatised chitosan was dissolved in the insulin by stirring using magnetic stirred at a low speed. The stirring was done for an hour. To seventy % of the oil or the fatty acid component, surfactant and HCl is added and stirred at 6000 rpm in a suitable preparation container using a high-speed stirrer using a half-moon paddle stirrer. After stirring for 15 minutes the insulin-polymer solution was added slowly without stopping the stirring. The stirring was continued for two hours at the same rpm. CaCl₂ 2H₂O is then dispersed in the remaining oil or fatty acid component by stirring vigorously using a magnetic stirrer. This dispersion is added after two hours to the oil-insulin-polymer mixture very slowly without stopping the stirring, which is then continued for again two hours at the same speed. The temperature of the system should be maintained at 35 to 36°C. The suspension is then filtered through a 100 nm micro filter and centrifuged at 10,000 rpm for twenty minutes. The supernatant is drained off and the pellet obtained is stored at 4-8°C.

### Example 2

**Materials**

| | |
|---|---|
| Alginic acid sodium salt | - 450.0 mg |
| Insulin solution (400 IU/ml) | - 5.0 ml |
| Phosphate buffer pH 7.0 (USP) | - 5.0 ml |
| Coconut oil | - 35.0 ml |
| Sorbitan monooleate- 80 | - 0.3-0.35 ml |
| N/10 HCl- | - 0.5 ml |
| Coconutoil : groundnutoil (7:3) | - 4.0-4.2 ml |
| CaCl₂ 2H₂O - | - 40.0 - 60.0 mg |
| ZnCl₂ - | - 9.0 -15.0 mg |

The Alginic acid sodium salt was dissolved in the insulin and phosphate buffer by stirring using magnetic stirrer at a low speed. The stirring was done for an hour. To 35 ml of the coconut oil, surfactant and HCl is added and stirred at 6000-7000 rpm in a suitable preparation container using a high-speed stirrer attached with a half-moon paddle stirrer. After stirring for 15 minutes the insulin-polymer solution was added slowly without stopping the stirring. The stirring was continued for one-two hours at the same rpm. Both CaCl₂ 2H₂O and ZnCl₂ are dispersed in the Coconutoil : groundnutoil (7:3) by stirring vigorously using a magnetic stirrer. This dispersion is added after one-two hours to the oil-insulin-polymer mixture very slowly without stopping the stirring, which is then continued for again 30 minutes to 2 hours at the same speed. The temperature of the system should be maintained at 35 to 36°C. The suspension is then filtered through a 100 nm micro filter and centrifuged at 10,000 rpm for twenty minutes. The supernatant is drained off and the pellet obtained is stored at 4-8°C.

### Example 3

**Materials**

| | |
|---|---|
| Derivatised Pullulan - 0.1- 0.9% optimized to get < 0.06g/g of the final product | |
| Insulin solution- | 10- 20% |
| Palm oil:Groundnutoil 20:80- | 75-78% |
| Sorbitan monooleate- | 0.8-1.0% |
| N/10 HCl- | 0.6 -1.2% |
| CaCl₂ 2H₂O- | 0.40- 0.60% |

### Example 4

| | |
|---|---|
| Alginic acid sodium salt- 0.1- 0.9% optimized to get < 0.06g/g of the final product | |
| Insulin solution | 10-20% |
| Fatty acid component | 75-80% |
| Surfactant | 0.4-0.8% |
| 0.01 N HCl | 0.8-1.2% |
| CaCl₂H₂O | 0.02-0.2% |
| ZnCl₂ | 0.02-0.08% |

The fatty acid component is a mixture of fatty acids. The fatty acids are Oleic acid, palmitic acid, myristic acid and lauric acid.

**Percentage of the fatty acids**

| | |
|---|---|
| Oleic acid | 5-20% |
| Palmitic acid | 5-20% |
| Lauric acid | 60-80% |
| Myristic acid | 0-10% |

The fatty acids are taken in such a way as to obtain the required volume for the process. The fatty acids are melted and the components are mixed to get a uniform fatty acid solution. An aliquot (10-30%) of the fatty acid solution is kept apart for dispersing crosslinking agents. To the remaining fatty acid solution surfactant and HCl is added and dispersed using a high speed stirrer at 1000-10000 rpm. After 10-30 minutes the insulin-polymer solution is added very slowly to the oil dispersion while stirring at the same speed. Stirring continued for one and half hours. Meanwhile the cross linking agents are dispersed in the fatty acid solution. The finely dispersed cross linking agents- in-fatty acid solution is then slowly added to the fatty acid-insulin-polymer dispersion without stopping the stirring. Stirring continued for another thirty minutes to two hours. The suspension is then filtered through a 100 nm filter paper; the nanoparticle pellet is collected by centrifugation at 10000 rpm for 20 minutes. The yield of nanoparticles is about 17 to 19 g % (w/v). It could be noted that the polymer material is only about 4-6% of the total nanoparticle formed. Here fatty acids form the major (>90%) component of the nanoparticles and the role of polymer is to incorporate stability, acts as a binder and imparts pH sensitivity to the nanoparticles. This nanoparticles is thus novel and unique and in the size range of <100 nm.

### Example 5

### In vivo experiments in diabetic rat model

The in vivo experiments were done on male Wistar rats. The rats were made diabetic using streptozotocin by giving an intraperitoneal injection at a dose of 50-mg/Kg-body weight of the rat.

The diabetic rats were orally given the nanoparticle placebo and insulin loaded nanoparticles at a dose of 3 and 6 IU/200 gm rat. A diabetic control was also maintained during the experiments.

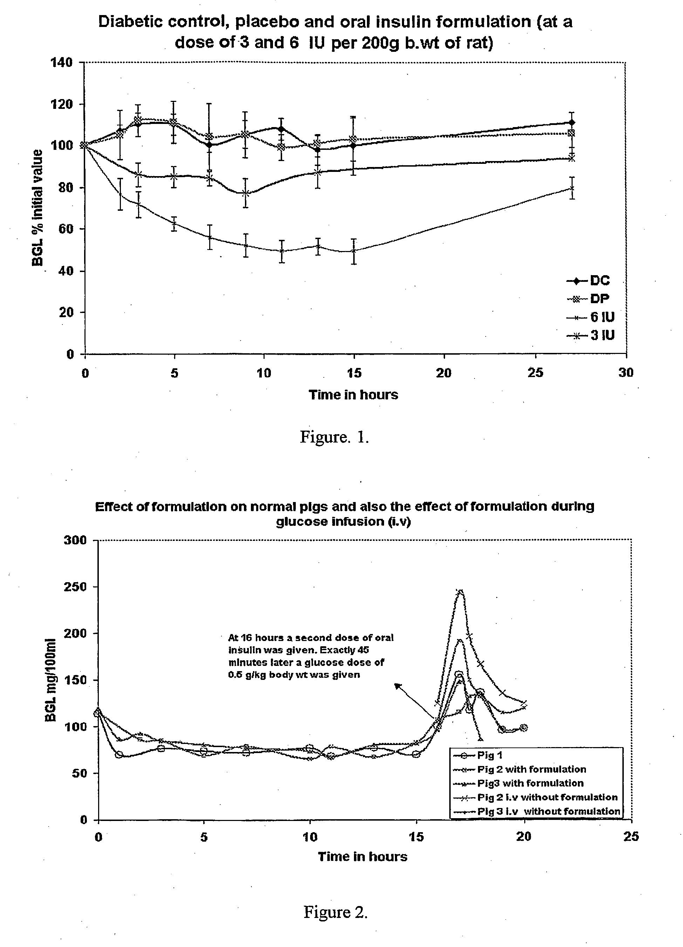

### Results are shown in Fig.1

Figure 1. Diabetic control, placebo and oral insulin formulation (at a dose of 3 and 6 IU/ 200 gm body weight of diabetic rat)

### Example 6

### In vivo experiments in normal pig

The in vivo experiments were done on normal pigs also. The effect of formulation was tested orally in normal conditions and also during intravenous glucose infusion. For assessing the efficacy of the formulation in presence of extra glucose, first the pigs were given an oral dose of insulin formulation. Then exactly 45 minutes later an intravenous glucose challenge was given (0.5g/Kg body wt.). In a previous experiment it was found that the peak value of glucose following an intravenous challenge occurs at 15' after glucose infusion. So the samples were collected at 15', 30', 60', 1, 2 and 3 hour. It was found that in oral insulin given pigs the peak of glucose after 15 minutes was reduced than in the pigs without formulation.

This results shows that the insulin-loaded nanoparticles when given orally is capable of reducing the blood glucose levels.

### Example 7

### In vivo experiments Diabetic pig model

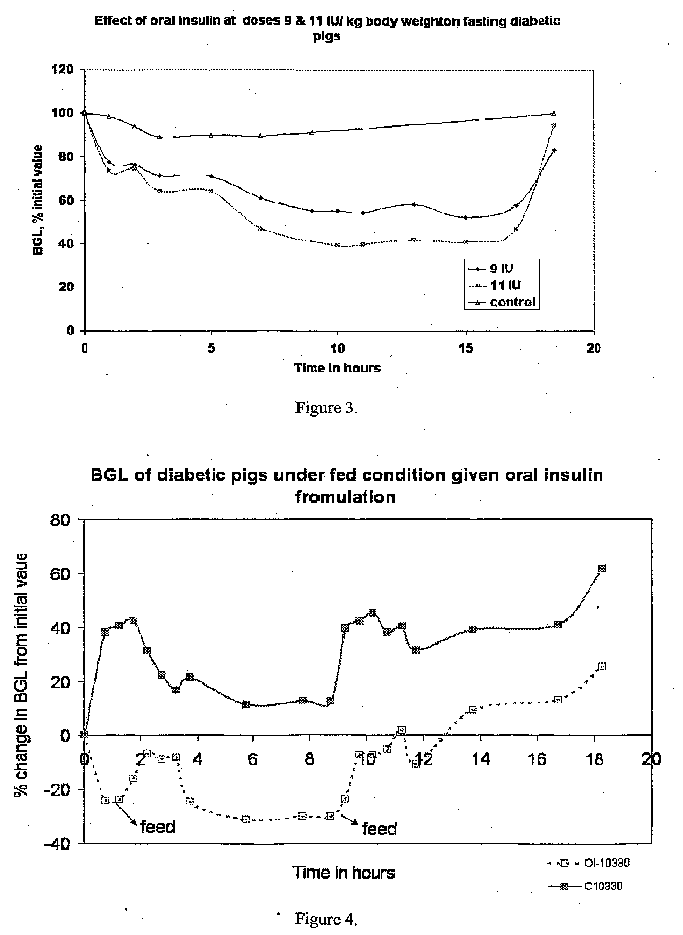

■ Male large white Yorkshire pigs were used to develop the diabetic pig model. Chronic catheterization - in jugular vein, Certofix central venous catheter - was the method adopted for continuous blood sampling.
■ Streptozotocin was given at a total dose of 190 mg/ kg body weight in 0.1 M sodium citrate buffer. Streptozotocin (STZ) was administered intravenously to the pigs under fasting conditions.
■ The blood glucose level was continuously monitored to prevent the hypoglycemia.
■ STZ was given as two doses (100 + 90 mg/kg body weight) at an interval of 48 hours.
■ Stability of the diabetic condition was established.
■ Insulin nanoparticles were given orally at two doses - 9 and 11 IU/ kg body weight under fasting conditions. (Figure no.3)
■ Effect of oral insulin formulation was studied in diabetic pigs under fed condition also. Pigs were given an oral dose of 20 IU/kg body weight and then feed was given. Blood glucose level was monitored. (figure no.4). The data shows the percentage changes in blood glucose level under fed conditions given oral insulin formulation (OI-10330) and without (C-10330) oral insulin formulation.

### Results are shown in Figures 2-4

Figure 2. Effect of formulation on normal pigs and also the effect of formulation during glucose infusion (i.v).
Figure3.. Effect of oral insulin formulation on fasting diabetic pigs at doses 9 & 11 IU/ kg body weight.
Figure 4. Effect of oral insulin formulation at a dose of 20 IU/kg body weight on BGL of diabetic pigs under fed conditions

### Example 8

### Peyer's patches experiment

Dye loaded nanoparticle was used for studying the mechanism of absorption of the nanoparticle via the Peyer's patches. The experiment was done to understand the gastrointestinal uptake of the nanoparticles. Fluorescein dye loaded nanoparticles was used for the study. Experiment was done on a normal albino Wistar rat. The rat was fasted for 20 hours with free access to water. The rat was anaesthetized using xylazine (6 mg/kg body wt.) by injecting intramuscularly. Under anesthesia the abdominal area of the rat was cleaned and the hair was removed. Then the abdomen was cut open by a midline incision to expose the intestine. At the beginning of the small intestine a small incision was made and a catheter was inserted which was then secured to the intestine using a cotton umbilical tape. At the end of the small intestine also a small incision was made and a catheter was inserted as mentioned above. A saline drip set was attached to one end and the whole intestinal segment was flushed out with normal saline. After flushing out the intestine a 20 ml aliquot of dye loaded nanoparticle suspension was infused. Both ends of the intestinal segment was then sealed by clamping the catheter using artery forceps. After two hours the dye solution was drained. The small intestinal segment was then washed out with 200 ml of normal saline.

The rat was then sacrificed by Occipital Atlantal dislocation. The intestinal tissue sections containing Peyer's patches were then collected in saline.The tissues were sectioned using a cryostat microtome and viewed using a fluorescent microscope with UV filter. It was proven that the nanoparticles are absorbed by Peyer's patches and as well as villi by the experiment as evidenced by the fluorescent microscopy photographs

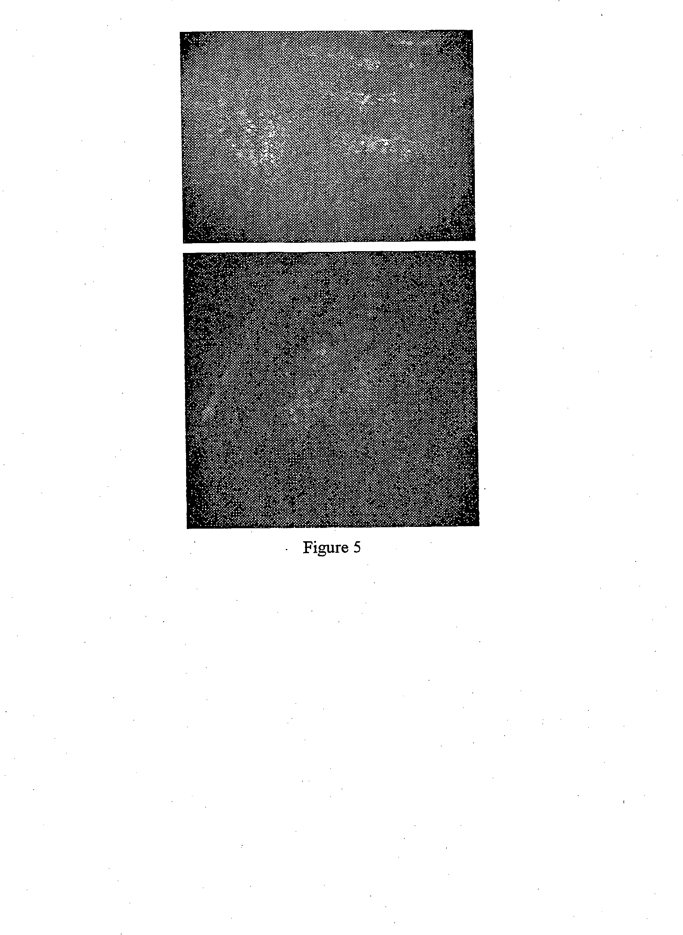

### Results are shown in Figure 5.

Figure 5. Nanoparticles in Peyer's patches and villi.

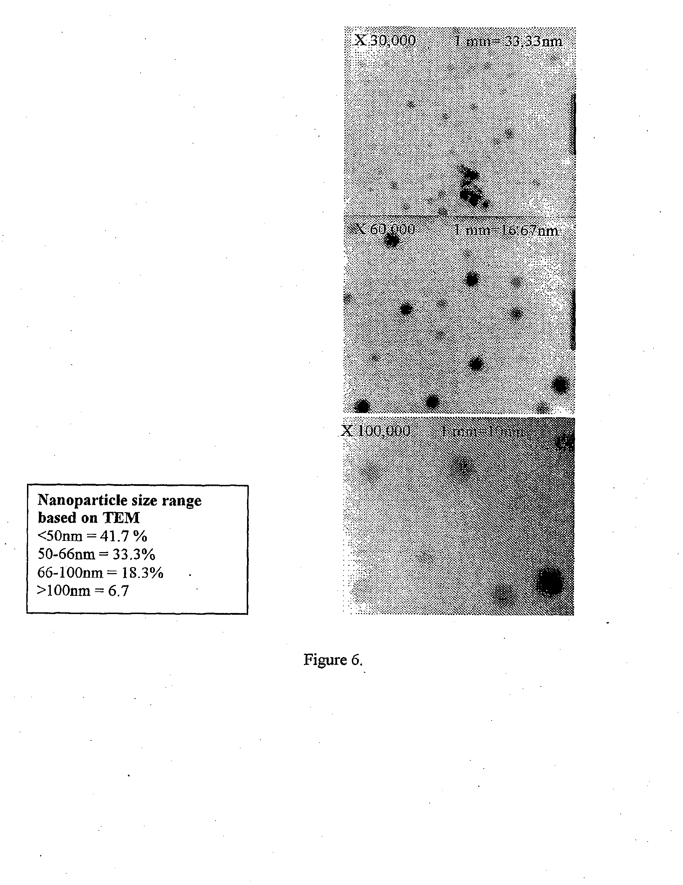

Figure6. Transmission Electron Photomicrograph of insulin loaded polymeric nanoparticles along with the size calculator.

## Claims

1. A pH sensitive nanoparticle formulation comprising:
a) polymer 4-6% (w/w), said polymer being selected from the group consisting of chitosin, alginate, pullulan, chitosan gellan, xanthan, poly methacrylic acid and derivatives thereof;
b) fatty acid 25-50% (w/w)
c) surfactant 0.5-5.0%(w/w)
d) protein 0.5-2.5%(w/w) (lmg of protein contains 25 IU), said protein being selected from insulin and hormones;
e) cross linking agent 0.02-0.3% (w/w)
f) water content 50-75% (w/w)

2. A pH sensitive nanoparticle formulation as claimed in claim 1 has the following characteristics:
a) having particle size distribution in the range of 30-100nm,
b) activity of the loaded insulin in the said formulation is 100% and is stable for a period of 5-7 months, at a temperature of about 4°C,
c) the said formulation is capable of lowering blood glucose level by over 50% in diabetic rats when administered with a dose of 6 IU/200 gm body weight,
d) bioavailability/absorption of protein is about 27% in the rat model when the said formulation is administered in the above said model.

3. A formulation as claimed in claim 1, wherein the cross linking agent used is selected from the group consisting of ZnCl₂, CaCl₂, glutaraldehyde and a mixture thereof.

4. A formulation as claimed in claim 1, wherein the fatty acid used is selected from the group consisting of lauric acid, oleic acid, palmitic acid, myristic acid and linoleic acid.

5. A formulation as claimed in claim 1, which is useful for oral delivery system in a body for the administration of peptide hormones and drugs.

6. A formulation as claimed in claim 1, which reduces blood glucose level by over 50% in diabetic rats when administered with a dose of 6 IU/200 gm body weight.

7. A process for the preparation of pH sensitive nanoparticle formulation comprising
a) polymer 4-6% (w/w), said polymer being selected from the group consisting of chitosin, alginate, pullulan, chitosan, gellan, xanthan, poly methacrylic acid and derivatives thereof;
b) fatty acid 25-50% (w/w)
c) surfactant 0.5-2.0%(w/w)
d) protein 0.5-2.5%(w/w) (lmg of protein contains 25 IU), said protein being selected from insulin and hormones;
e) cross linking agent 0.02-0.3% (w/w)
f) water content 50-75% (w/w)
the said process comprises the steps of:
a) preparing a solution of 0.1-0.9% polymer and 40-400IU/mL protein (20% v/v) in buffer, followed by stirring the above said solution mixture, for a period of about 1 hr,
b) preparing an oil suspension of 0.2-1.0% surfactant and 0.5-2.0% HCl (0.1N) in oil or fatty acid, under stirring at 6000-7000 rpm, over a period of 10-20 min, at a temperature of 30-35°C,
c) preparing a cross linking solution of 0.005-0.3%(w/w) cross linking agent in oil or fatty acid, under stirring vigorously over a period of about 1 hr,
d) adding soluble polymer-protein solution mixture obtained in step (a) to an oil suspension obtained in step (b) under stirring, for a period of about 2 hrs,
e) adding drop wise cross linking solution obtained in step (c) to a solution mixture obtained in step (d), under stirring, and continuing the stirring for a period of about 2 hrs, at 600-700 rpm , at a temperature of 30-35°C,
f) filtering the above said solution mixture through 100 nm micro filter, followed by centrifugation at about 10,000 rpm, for a period of 20-30 min and draining the supernatant to obtained the desired nano particle formulation.

8. A process as claimed in claim 7, wherein the cross linking agent used is selected from the group consisting of ZnCl₂, CaCl₂, glutaraldehyde and a mixture thereof.

9. A process as claimed in claim 7, wherein the fatty acid used is selected from the group consisting of lauric acid, oleic acid myristic acid, palmitic acid and linoleic acid.

10. A process as claimed in claim 7, wherein the said pH sensitive nanoparticle formulation obtained has the following characteristics:
a) having the particle size distribution as prepared for TEM analysis is in the range of 30-100nm (>90%),
b) activity of the loaded insulin in the said formulation is 100% and is stable for a period of 5-7 months, at a temperature of about 4°C,
c) the said formulation is capable of lowering blood glucose level by over 50% in diabetic rats when administered with a dose of 6 IU/200 gm body weight,
d) bioavailability/absorption of protein is about 27% in the rat model when the said formulation is administered in the above said model.

## Patentansprüche

1. PH-empfindliche nanoteilchenförmige Formulierung, umfassend:
a) Polymer 4 bis 6 % (Massenprozent), wobei dieses Polymer aus der Gruppe, bestehend aus Chitosin, Alginat, Pullulan, Chitosan, Gellan, Xanthan, Poly-Methacrylsäure und deren Derivate ausgewählt ist;
b) Fettsäure 25 bis 50 % (Massenprozent);
c) Tensid 0,5 bis 5,0 % (Massenprozent);
d) Protein 0,5 bis 2,5 % (Massenprozent) (1 mg von Protein enthält 25 IU), wobei das Protein aus Insulin und Hormonen ausgewählt ist;
e) Vernetzungsmittel 0,02 bis 0,3 % (Massenprozent);
f) Wassergehalt 50 bis 75 % (Massenprozent).

2. PH-empfindliche nanoteilchenförmige Formulierung nach Anspruch 1, aufweisend folgende Eigenschaften:
a) aufweisend eine Teilchengrößenverteilung im Bereich 30 bis 100 nm;
b) die Aktivität des in diese Formulierung eingebrachten Insulins beträgt 100 % und ist für einen Zeitraum von 5 bis 7 Monaten bei einer Temperatur von zirka 4 °C stabil;
c) diese Formulierung ist in der Lage, den Blutzuckergehalt bei an Diabetes leidenden Ratten um über 50 % zu senken, wenn sie in einer Dosierung von 6 IU/200 g Körpergewicht verabreicht wird;
d) die biologische Verfügbarkeit/Absorption des Proteins beträgt zirka 27 % beim Rattenmodell, wenn die Formulierung dem genannten Rattenmodell verabreicht wird.

3. Formulierung nach Anspruch 1, wobei das eingesetzte Vernetzungsmittel aus der Gruppe, bestehend aus ZnCl₂, CaCl₂, Glutaraldehyd und einer Mischung daraus, ausgewählt wird.

4. Formulierung nach Anspruch 1, wobei die eingesetzte Fettsäure aus der Gruppe, bestehend aus Laurinsäure, Ölsäure, Palmitinsäure, Myristinsäure und Leinölsäure, ausgewählt ist.

5. Formulierung nach Anspruch 1, die nützlich zur oralen Abgabe in einen Körper zur Verabreichung von Peptidhormonen und -Medikamenten ist.

6. Formulierung nach Anspruch 1, die den Blutzuckergehalt bei an Diabetes leidenden Ratten um über 50 % senkt, wenn sie in einer Dosierung von 6 IU/200 g Körpergewicht verabreicht wird.

7. Verfahren zur Herstellung einer pH-empfindlichen nanoteilchenförmigen Formulierung, umfassend
a) Polymer 4 bis 6 % (Massenprozent), wobei dieses Polymer aus der Gruppe, bestehend aus Chitosin, Alginat, Pullulan, Chitosan, Gellan, Xanthan, Poly-Methacrylsäure und deren Derivate ausgewählt ist;
b) Fettsäure 25 bis 50 % (Massenprozent);
c) Tensid 0,5 bis 2,0 % (Massenprozent);
d) Protein 0,5 bis 2,5 % (Massenprozent) (1 mg von Protein enthält 25 IU), wobei das Protein aus Insulin und Hormonen ausgewählt ist;
e) Vernetzungsmittel 0,02 bis 0,3 % (Massenprozent);
f) Wassergehalt 50 bis 75 % (Massenprozent).
wobei das Verfahren folgende Schritte umfasst:
a) Herstellung einer Lösung aus 0,1 bis 0,9 % Polymer und 40 bis 400 IU/ml Protein (20 % Volumenprozent im Puffer, gefolgt vom Rühren dieser Lösungsmischung für einen Zeitraum von zirka 1 Stunde;
b) Herstellung einer Ölsuspension aus 0,2 bis 1,0 % Tensid und 0,5 bis 2,0 % HCl (0.1N) in Öl- oder Fettsäure unter Rühren bei einer Drehzahl von 6000 bis 7000 U/min über einen Zeitraum von 10 bis 20 Minuten bei einer Temperatur von 30 bis 35 °C;
c) Herstellung einer Vernetzungslösung aus 0,005 bis 0,3 % (Massenprozent) Vernetzungsmittel in Öl- oder Fettsäure unter kräftigem Rühren über einen Zeitraum von zirka 1 Stunde;
d) Hinzufügen der löslichen Polymer-Protein-Lösungsmischung aus Schritt (a) zu einer Ölsuspension aus Schritt (b) unter Rühren für einen Zeitraum von zirka 2 Stunden;
e) tropfenweises Hinzufügen der Vernetzungslösung aus Schritt (c) zu einer Lösungsmischung aus Schritt (d) unter Rühren und Weiterrühren für einen Zeitraum von zirka 2 Stunden bei 600 bis 700 U/min und einer Temperatur von 30 bis 35 °C;
f) Filtern der Lösungsmischung durch einen 100-nm-Mikrofilter, gefolgt vom Zentrifugieren bei zirka 10.000 U/min für einen Zeitraum von 20 bis 30 Minuten und Abführen des Tensids, um die gewünschte nanoteilchenförmige Formulierung zu erhalten.

8. Verfahren nach Anspruch 7, wobei das eingesetzte Vernetzungsmittel aus der Gruppe, bestehend aus ZnCl₂, CaCl₂, Glutaraldehyd und einer Mischung daraus, ausgewählt wird.

9. Verfahren nach Anspruch 7, wobei die eingesetzte Fettsäure aus der Gruppe, bestehend aus Laurinsäure, Ölsäure, Palmitinsäure, Myristinsäure und Leinölsäure, ausgewählt ist.

10. Verfahren nach Anspruch 7, wobei die erhaltene pH-empfindliche nanoteilchenförmige Formulierung folgende Eigenschaften aufweist:
a) aufweisend die Teilchengrößenverteilung gemäß der Vorbereitung für die TEM-Analyse im Bereich 30 bis 100 nm (>90 %);
b) die Aktivität des in diese Formulierung eingebrachten Insulins beträgt 100 % und ist für einen Zeitraum von 5 bis 7 Monaten bei einer Temperatur von zirka 4 °C stabil;
c) diese Formulierung ist in der Lage, den Blutzuckergehalt bei an Diabetes leidenden Ratten um über 50 % zu senken, wenn sie in einer Dosierung von 6 IU/200 g Körpergewicht verabreicht wird;
d) die biologische Verfügbarkeit/Absorption des Proteins beträgt zirka 27 % beim Rattenmodell, wenn die Formulierung dem genannten Rattenmodell verabreicht wird.

## Revendications

1. Formulation de nanoparticules sensibles au pH comprenant :
a) un polymère à 4-6 % (M/M), ledit polymère étant choisi dans le groupe constitué par la chitosine, l'alginate, le pullulan, le chitosan, le gellane, le xanthane, l'acide polyméthacryle et leurs dérivés ;
b) un acide gras à 25-50 % (M/M)
c) un tensioactif à 0,5-5,0 %(M/M)
d) une protéine à 0,5-2,5 % (M/M) (1 mg de protéine contient 25 U.I.), ladite protéine étant choisie parmi l'insuline et les hormones ;
e) un agent de réticulation à 0,02-0,3 % (M/M) ;
f) une teneur en eau à 50-75 % (M/M).

2. Formulation de nanoparticules sensibles au pH selon la revendication 1 ayant les caractéristiques suivantes :
a) une granulométrie comprise entre 30 et 100 nm,
b) une activité de l'insuline chargée dans ladite formulation à 100 % et stable pendant une durée comprise entre 5 et 7 mois, à une température d'environ 4° C,
c) ladite formulation est en mesure d'abaisser le taux de glycémie dans le sang de plus de 50 % sur des rats diabétiques lorsqu'elle est administrée avec une dose de 6 U.I./200 g de poids corporel,
d) la biodisponibilité/absorption de la protéine est d'environ 27 % chez le rat lorsque ladite formulation est administrée dans le modèle susmentionné.

3. Formulation selon la revendication 1, dans laquelle l'agent de réticulation utilisé est choisi dans le groupe constitué par le ZnCl₂, le CaCl₂, le glutaraldéhyde et leur mélange.

4. Formulation selon la revendication 1, dans laquelle l'acide gras utilisé est choisi dans le groupe constitué par l'acide laurique, l'acide oléique, l'acide palmitique, l'acide myristique et l'acide linoléique.

5. Formulation selon la revendication 1, étant utile pour un mode d'administration orale dans un corps pour l'administration d'hormones et médicaments peptidiques.

6. Formulation selon la revendication 1, réduisant le taux de glycémie dans le sang de plus de 50 % sur des rats diabétiques lorsqu'elle est administrée avec une dose de 6 U.I./200 g de poids corporel.

7. Procédé destiné à la préparation d'une formulation de nanoparticules sensibles au pH comprenant :
a) un polymère à 4-6 % (M/M), ledit polymère étant choisi dans le groupe constitué par la chitosine, l'alginate, le pullulan, le chitosan, le gellane, le xanthane, l'acide polyméthacryle et leurs dérivés ;
b) un acide gras à 25-50 % (M/M)
c) un tensioactif à 0,5-2,0%(M/M)
d) une protéine à 0,5-2,5 % (M/M) (1 mg de protéine contient 25 U.I.), ladite protéine étant choisie parmi l'insuline et les hormones ;
e) un agent de réticulation à 0,02-0,3 % (M/M) ;
f) une teneur en eau à 50-75 % (M/M) ;
ledit procédé comprend les étapes de :
a) préparer une solution de polymère à 0,1-0,9 % et de protéine de 40-400 U.I./mL (20% v/v) par régulation, puis agiter ledit mélange de solution susmentionné pendant une heure environ,
b) préparer une suspension d'huile de tensioactif à 0,2-1,0 % et de HCL à 0,5-2,0 % (0,1 N) dans de l'huile ou de l'acide gras, sous agitation à 6000-7000 t/mn, pendant 10 à 20 minutes, à une température comprise entre 30 et 35 °C,
c) préparer une solution réticulaire d'un agent de réticulation à 0,005-0,3 % (M/M) dans de l'huile ou de l'acide gras, sous agitation énergique pendant environ une heure,
d) ajouter le mélange de solution polymère/protéine obtenue à l'étape (a) à une suspension d'huile obtenue à l'étape (b) sous agitation, pendant environ 2 heures,
e) ajouter la solution réticulaire obtenue à l'étape (c) à un mélange de solution obtenue à l'étape (d) goutte à goutte, sous agitation et continuer l'agitation pendant environ 2 heures à 600-700 t/mn, à une température comprise entre 30 et 35° C,
f) filtrer ledit mélange de solution susmentionné à travers un filtre au micron de 100 nm, puis par centrifugation à environ 10 000 t/mn pendant 20 à 30 minutes et drainer le surnageant pour obtenir la formulation de nanoparticules désirée.

8. Procédé selon la revendication 7, dans lequel l'agent de réticulation utilisé est choisi dans le groupe constitué par le ZnCl₂, le CaCl₂, le glutaraldéhyde et leur mélange.

9. Procédé selon la revendication 7, dans lequel l'acide gras utilisé est choisi dans le groupe constitué par l'acide laurique, l'acide oléique, l'acide myristique, l'acide palmitique et l'acide linoléique.

10. Procédé selon la revendication 7, dans lequel ladite formulation de nanoparticules sensibles au pH obtenue possède les caractéristiques suivantes :
a) une granulométrie, telle que préparée pour une analyse au MET,
comprise entre 30 et 100 nm (> 90 %),
b) une activité de l'insuline chargée dans ladite formulation à 100 % et stable pendant une durée comprise entre 5 et 7 mois à une température d'environ 4° C,
c) ladite formulation est en mesure d'abaisser le taux de glycémie dans le sang de plus de 50 % sur des rats diabétiques lorsqu'elle est administrée avec une dose de 6 U.I./200 g de poids corporel,
d) une biodisponibilité/absorption de la protéine d'environ 27 % chez le rat lorsque ladite formulation est administrée dans le modèle susmentionné.
